# EUROPEAN PATENT SPECIFICATION

(11) **EP 2 909 330 B1**
(45) Date of publication and mention of the grant of the patent: **07.04.2021**
(21) Application number: 13847108.1
(22) Date of filing: 09.10.2013
(51) Int. Cl.: C12Q 1/02, C12N 1/16, G01N 21/64, G01N 15/14, G01N 15/00

(54) **AUTOMATED YEAST BUDDING MEASUREMENT**
AUTOMATISIERTE MESSUNG DER HEFEZELLENKNOSPUNG
MESURE AUTOMATISÉE DU BOURGEONNEMENT DE LEVURE

(30) Priority: 18.10.2012 US 201261715496 P
(43) Date of publication of application: 26.08.2015
(73) Proprietor: Nexcelom Bioscience LLC, Lawrence, MA 01843 (US)
(72) Inventor: CHAN, Leo, L., North Andover, MA 01845 (US); QIU, Jean, Andover, MA 01810 (US); LI, Peter, Andover, MA 01810 (US); FLANAGAN, Kevin, Sterling, MA 01564 (US)
(74) Representative: Ter Meer Steinmeister & Partner
(86) International application number: PCT/US2013/064003
(87) International publication number: WO 2014/062431

(56) References cited:
- WO-A2-2011/156249
- WO-A2-2011/156249
- Meredith E.K. Calvert ET AL: "Yeast Cell Cycle Analysis: Combining DNA Staining with Cell and Nuclear Morphology" In: "Current Protocols in Cytometry", April 2001 (2001-04), John Wiley & Sons, Inc., US, XP55254427, ISSN: 1934-9297 pages 9.32.1-9.32.16, DOI: 10.1002/0471142956.cy0932s52, * page 9.32.1 - page 9.32.10 * * *
- LEO L CHAN ET AL: "Direct concentration and viability measurement of yeast in corn mash using a novel imaging cytometry method", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 38, no. 8, 20 October 2010 (2010-10-20), pages 1109-1115, XP019928932, ISSN: 1476-5535, DOI: 10.1007/S10295-010-0890-7
- LAVERTY DANIEL J ET AL: "Automated quantification of buddingSaccharomyces cerevisiaeusing a novel image cytometry method", JOURNAL OF INDUSTRIAL MICROBIOLOGY AND BIOTECHNOLOGY, BASINGSTOKE, GB, vol. 40, no. 6, 5 April 2013 (2013-04-05), pages 581-588, XP035330699, ISSN: 1367-5435, DOI: 10.1007/S10295-013-1263-9 [retrieved on 2013-04-05]
- MEREDITH E.K. CALVERT E ET AL.: 'Yeast cell cycle analysis: combining DNA staining with cell and nuclear morphology.' CURRENT PROTOCOLS IN CYTOMETRY 2010, pages 9.32.1 - 9.32.16, XP055254427
- HELD PAUL PH.D.: 'Monitoring growth of beer brewing strains of Saccharomyces cerevisiae' 2010, pages 1 - 6, XP055255096 Retrieved from the Internet: <URL:http://www.biotek.com/assets/tech resources/SynergyHl Yeast Growth App Note. pdf>
- DEMIRBAS AYHAN.: 'Biofuels sources, biofuel policy, biofuel economy and global biofuel projections' ENERGY CONVERSION AND MANAGEMENT vol. 49, 2008, pages 2106 - 2116, XP022694945

## Description

### Technical Fields of the Invention

The invention generally relates to analysis and measurement of yeast cells. More particularly, the invention relates to efficient and effective methods for assessing and measuring yeast budding, viability and concentration of yeast cells.

### Background of the Invention

The biofuel and brewery industries have been utilizing baker's yeast (*Saccharomyces cerevisiae*) as the primary organism to commence fermentation process that produces CO₂ and bioethanol for their products. Currently, the largest biofuel process relies heavily on ethanol production, which utilizes *Saccharomyces cerevisiae* to perform fermentation on sugar cane, corn meal, polysaccharides, and waste water. Due to their high ethanol tolerance, final ethanol concentration, glucose conversion rate, and the historical robustness of industrial fermentation, yeasts are the ideal component for bioethanol production. (Antoni, et al. 2007 Appl. Microbiol. Biotech. vol. 77, pp. 23-35; Vertès, et al. 2008 J. Mol. Microbiol. and Biotech. vol. 15, pp. 16-30; Basso, et al. 2008 FEMS Yeast Res. vol. 8, pp. 1155-1163; Nikolic, et al. 2009 J. Chem. Technolog. Biotech. vol. 84, pp. 497-503; Gibbons, et al. 2009 In Vitro Cell. & Developm. Biol. - Plant, vol. 45, pp. 218-228; Hu, et al. 2007 Genetics, vol. 175, pp. 1479-1487; Argueso, et al. 2009 Genome Res. vol. 19, pp. 2258-2270; Eksteen, et al. 2003 Biotech. and Bioeng. vol. 84, pp. 639-646.)

Yeast budding is one of the most important parameters that breweries and biofuel companies use to determine the quality of the fermentation. Yeast pitching time for propagation and fermentation is the percentage of budding yeasts in the sample, which is known to estimate growth rate of yeast. Currently, there is no existing simple automated yeast budding detection method. Image flow cytometers have been used to perform yeast cell cycle to measure budding percentages. (Meredith, et al. 2008 Cytometry Part A, 73A: 825-833.) Image flow cytometers, however, are relatively expensive and require considerable amount of maintenance as well as highly trained technician for operation. Therefore, it is not suited for quality assurance in an industrial production setting. In addition, flow based sample preparation does not work with the biofuel samples due to the large corn mash debris in the sample that would clog the fluidics in the system.

Conventional analytical methods for concentration, viability and yeast budding percentages involve manual counting of yeasts particles in a hemacytometer under conventional light microscopy and colony counting of colony forming units in plating. These methods are tedious and time-consuming and are inherently inconsistent due to operator subjectivity. In order to obtain an accurate representation of the behavior of yeast during fermentation, an automated method for measuring concentration, viability, and budding percentage of the sample are required.

Meredith E. K. Calvert et al: "Yeast Cell Cycle Analysis: Combining DNA Staining with Cell and Nuclear Morphology", in: "Current Protocols in Cytometry", April 2010, John Wiley & Sons, Inc., US, pp. 9.32.1-9.32.16 is related to a study demonstrating the combined use Of DNA content measurements and bright-fields image analysis by using multispectral imaging flow cytometry (MIFC) for quantification of yeast cell cycle distribution. Leo L Chan et al.: "Direct concentration and viability measurement of yeast in corn mash using a novel imaging cytometry method", Journal of Industrial Microbiology & Biotechnology, ; Official Journal of the Society for industrial Microbiology, Springer, Berlin, DE, vol. 38, no. 8, 20 October 2010, pp. 1109-1115 is related to an imaging cytometry method for concentration and viability measurements of yeast in corn mash from operating fermenters. WO 2011/156249 A2 is related to analyzing yeast viability, in particular, to methods and compositions for accessing and measuring viability and concentration of yeast cells.

Therefore, there is an unmet need for an automated method for accurate and efficient measurement of yeast concentration, viability and budding percentage.

### Summary of the Invention

The invention is based, in part, on the discovery of efficient and effective methods for automated measurement of yeast budding percentage. The present invention addresses the shortcomings of the previous methods in that real-time samples such as those from biofuel and wine production plants may be readily and accurately analyzed by the methods of the invention. Messy samples can be effectively measured as the invention allows high staining specificity. The automated, image-based cytometry method of the invention greatly simplifies the measurement process for the biofuel and brewery industries because it allows quick, accurate and concurrent determination of yeast budding, concentration and viability.

Described herein is a method for automated analysis of budding status of yeast cells. The method includes: staining a sample to be analyzed for yeast cell budding with a dye in a buffer solution; acquiring a fluorescent image of the dye-stained sample; analyzing the fluorescent image of the dye-stained sample to determine the aspect ratio of the images of yeast cells in the dye-stained sample by a computer-based automated process, thereby determining the status of budding yeast cells in the sample.

In one aspect, the invention is directed to a method for simultaneously determining yeast budding status and viability of yeast cells in a sample as defined in claim 1.

In another aspect, the invention is directed to a method for simultaneously determining yeast budding status, viability, and concentration of yeast cells in a sample as defined in claim 6.

### Brief Description of the Drawings

**FIG. 1** depicts certain exemplary results from an embodiment of the method according to the invention, showing imaging analysis method for enumerating budding yeasts.
**FIG. 2** depicts certain exemplary results from an embodiment of the method according to the invention, showing budding percentages measured from a growing yeast population.
**FIG. 3** depicts certain exemplary results from an embodiment of the method according to the invention, showing comparisons with traditional manual counting method.

### Detailed Description of the Invention

The present invention addresses the shortcomings of the previous methods and provides real-time and accurate analysis on a variety of samples such as those from biofuel plants that contain corn mash and other debris. Due to the high staining specificity, messy samples can be effectively measured. The invention also offers great efficiency and effectiveness by allowing simultaneous analysis and measurement of viability and concentration of yeast cells.

Recently, a novel imaging cytometry method has been developed by Nexcelom Bioscience (Lawrence, MA), which allows rapid measurement of cell concentration using inexpensive disposable counting chambers that require only 20 µl of samples. (Lai, et al. 2009 J. Clin. Oncology vol. 27, pp. 1235-1242; Nott, et al. 2009 J. Biol. Chem. vol. 284, pp. 15277-15288; Qiao, et al. 2009 Arteriosclerosis Thrombosis and Vascular Biol. vol. 29, pp. 1779-U139; Rounbehler, et al. 2009 Cancer Res. vol. 69, pp. 547-553; Shanks, et al. 2009 Appl. and Envir.Microbiol. vol. 75, pp. 5507-5513; Stengel, et al. 2009 Endocrinology, vol. 150, pp. 232-238.)

Utilizing combined bright-field and fluorescent imaging, the system allows automated cell image acquisition and processing using a novel counting algorithm for accurate and consistent measurement of cell population and viability on a variety of cell types. Applications such as enumeration of immunological, cancer, stem, insect, adipocytes, hepatocytes, platelets, algae, and heterogeneous cells, quantification of GFP transfection, viability using Trypan Blue or Propidium Iodide, measuring WBCs in whole blood, have been previously reported. More importantly, the method has been shown to produce consistent concentration and viability measurements of pure yeast for quality control purposes in biofuel, beverage, and baking industry. (Nexcelom Bioscience, "Simpe, Fast and Consistent Determination of Yeast Viability using Oxonol," in Application Focus: Cellometer Vision 10X, pp. 1-2.)

Disclosed herein is a novel imaging fluorescence cytometry method employing the Cellometer® Vision (Nexcelom Bioscience, Lawrence, MA) for determining yeast budding, concentration and viability, for example, in corn mash from operating fermenters. Using a dilution buffer and staining the sample with Acridine Orange (AO) and Propidium Iodide (PI), the budding status, viable and nonviable yeasts are selectively labeled while nonspecific fluorescent signals from corn mash are eliminated. This method can efficiently perform yeast quality control using samples directly from processing fermenters without further filtration treatment, which can have a dramatic impact on monitoring consistent bioethanol production in the United States. Besides corn mash, viability of yeast in sugar cane fermentation can also be measured using this method. The method can also be readily applied to quality control in brewery production processes.

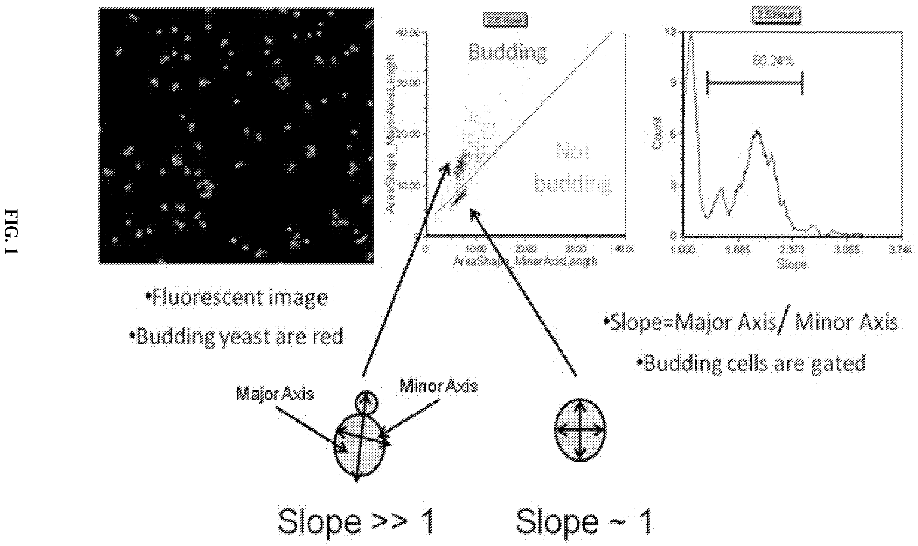

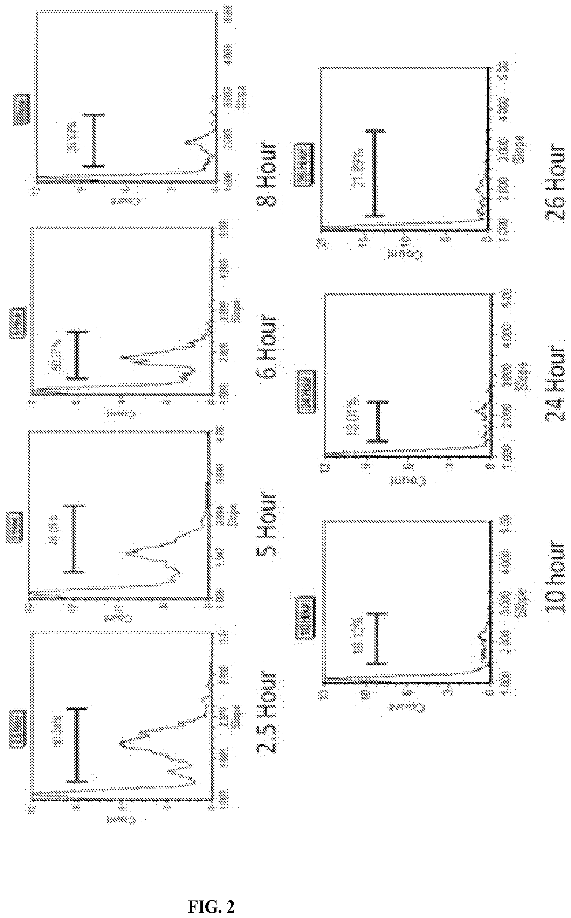

As depicted in **FIG. 1****,** the invention utilizes a system that includes an automated microscopy, fluorescent stains and buffer, and an image analysis method. The image analysis aspect of the invention utilizes captured images of fluorescently stained-yeasts, and measures the major and minor axis length of each yeast particle. The ratio of major to minor axis length, defined herein as "slope = major/minor", provides a variable (parameter) by which the budding status of yeast can be assessed. For instance, if the yeast particle is budding, then the major axis length will be greater than the minor axis length, thus producing a slope value greater than 1, whereas if the yeast particle is non-budding, the slope is to have a value of about 1 for a round shaped yeast. Using this parameter one can automatically gate the second population in **FIG. 2** as the budding population.

Described herein is a method for automated analysis of budding status of yeast cells. The method includes: staining a sample to be analyzed for yeast cell budding with a dye in a buffer solution; acquiring a fluorescent image of the dye-stained sample; analyzing the fluorescent image of the dye-stained sample to determine the aspect ratio of the images of yeast cells in the dye-stained sample by a computer-based automated process, thereby determining the status of budding yeast cells in the sample.

In certain preferred embodiments, the computer-based automated process includes automated measurement of the shape of budding yeasts in the sample. The threshold may be set such that a yeast cell (normally round shaped) is considered budding if its aspect ratio is 1.1 or greater. Other threshold may be set dependent on the application, for example at aspect ratio of 1.15 or greater, 1.2 or greater, 1.25 or greater, etc.

The dye may be any dye suitable for staining and analysis, for example, one or more selected from selected from the group consisting of Acridine Orange, SYTO 9, DAPI, Hoechst, Calcofluor White, Propidium Iodide, Ethidium Bromide, Oxonol, Mg-ANS, Acriflavine, ConA-FITC. The amount/concentrations of dyes used are dependent on the applications at hand. In the case of Acridine Orange, for example, a concentration may be in the range from about 1 µg/mL to about 50 µg/mL (e.g., about 2 µg/mL to about 50 µg/mL, about 5 µg/mL to about 50 µg/mL, about 10 µg/mL to about 50 µg/mL, about 20 µg/mL to about 50 µg/mL, about 25 µg/mL to about 50 µg/mL, about 1 µg/mL to about 40 µg/mL, about 1 µg/mL to about 30 µg/mL, about 1 µg/mL to about 20 µg/mL, about 1 µg/mL to about 10 µg/mL).

The buffer may be any suitable buffer solution, for example, with a pH in the range from about 5 to about 12 (*e.g.,* in a range from about 6 to about 12, from about 7 to about 12, from about 8 to about 12, at about 8, 9, 10, 11 or 12).

Any suitable samples may be analyzed by the method disclosed herein. For example, the sample may be one from a process of alcohol production using yeast. In certain embodiments, the sample to be tested is a sample from a biofuel fermentation process. The sample to be tested may contain certain debris, such as one or more of corn mash, sugar cane, cellulose and corn stover.

The methods of the invention is suitable for analyzing and measuring samples from the biofuel fermentation process producing one or more of ethanol, butanol and methanol from biomass.

Other examples of samples suitable for analysis by the disclosed methods include samples from a wine production process.

The methods are generally suitable for measuring budding status of yeast in general. Exemplary species of yeast include *Saccharomyces cerevisiae.*

In an aspect, the invention is directed to a method for simultaneously determining yeast budding status and viability of yeast cells in a sample as defined in claim 1.

In the case of Acridine Orange, for example, a concentration may be in the range from about 1 µg/mL to about 50 µg/mL (e.g., about 2 µg/mL to about 50 µg/mL, about 5 µg/mL to about 50 µg/mL, about 10 µg/mL to about 50 µg/mL, about 20 µg/mL to about 50 µg/mL, about 25 µg/mL to about 50 µg/mL, about 1 µg/mL to about 40 µg/mL, about 1 µg/mL to about 30 µg/mL, about 1 µg/mL to about 20 µg/mL, about 1 µg/mL to about 10 µg/mL).Also in the case of Propidium Iodide, for example, a concentration may be in the range from about 1 µg/mL to about 50 µg/mL (e.g., about 2 µg/mL to about 50 µg/mL, about 5 µg/mL to about 50 µg/mL, about 10 µg/mL to about 50 µg/mL, about 20 µg/mL to about 50 µg/mL, about 25 µg/mL to about 50 µg/mL, about 1 µg/mL to about 40 µg/mL, about 1 µg/mL to about 30 µg/mL, about 1 µg/mL to about 20 µg/mL, about 1 µg/mL to about 10 µg/mL).

The first dye is selected from the group consisting of Acridine Orange, SYTO 9, DAPI, Hoechst, Calcofluor White and the second dye is selected from the group consisting of Propidium Iodide, Ethidium Bromide, Oxonol, Mg-ANS. In certain preferred embodiments, the first dye is Acridine Orange and the second dye is Propidium Iodide.

In another aspect, the invention is directed to a method for simultaneously determining yeast budding status, viability, and concentration of yeast cells in a sample as defined in claim 6.

The developed automated yeast budding detection method can be applied to numerous type of yeasts. The measured slope parameter can be adjusted so that the restriction on the size of the bud can be fixed to remove the subjectivity between different technicians. This disclosed method is rapid and simple and can be easily adapted to a quality assurance setting at production or research facilities for the brewery and biofuel industries. Further adding to the uniqueness of the disclosed invention is that all three important parameters (yeast concentration, viability and budding percentages) can be measured simultaneously.

### Examples

### Yeast Preparation

A yeast growth culture was prepared by incubating yeast in YPD medium overnight at 30 °C. The yeast culture (800 µL) was then re-suspended in a 20 mL medium glass tube by shaking at 30 °C. The yeasts were collected at time points: 2.5, 5, 6, 8, 10, 24, and 30 hours and were stained with Acridine Orange and Propidium Iodide. The fluorescent images were captured.

### Automated Detection

At each time point, the fluorescent images were analyzed using Cell Profiler (Cambridge, MA) and Nexcelom Cellometer Software (Lawrence, MA), where the exported data was imported into FCS Express 4 Image (Los Angeles, CA). The FCS Express 4 was then used to plot the slope of each yeast particle so that the two populations (budding and non-budding) are separated and measured (**FIG**. **1**). This method was incorporated into the Cellometer® software so that the slope value was used to determine budding percentages, while the concentration and viability were measured simultaneously.

### Manual Comparison

At each time point, manual counting of yeast particles and budding are performed under bright-field imaging and fluorescent imaging. Total yeast particles and yeasts that are budding are manual counted to generate the budding percentage in the sample. The criterion was currently set that if two yeasts were touching, then it would be counted as one bud. The results of the manual counting were compared to the automated detection method.

### Validation of Automated Method

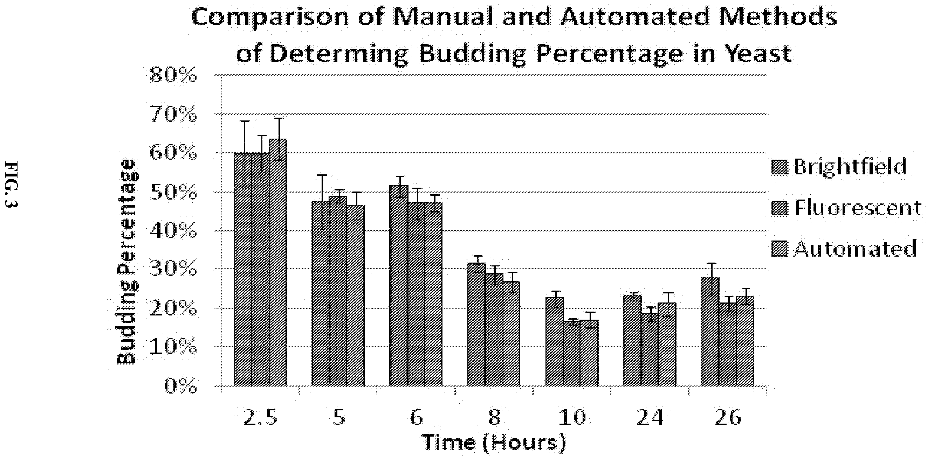

The gating results for the automated budding detection method at each time point are shown in **FIG. 3****.** There was a clear trend where, in the beginning of the growth phase, high percentages of budding were observed. Then from the lag phase, log phase to station phase, the budding percentages decreased. The budding percentages decreased from ∼60 to 20% during the growth period.

The automated budding results were compared to the bright-field and fluorescent manual counting (**FIG**. **3**). The results showed comparable budding percentages measured between all 3 methods. The bright-field manual counting typically overestimate the budding due to counting as debris, whereas the fluorescent manual counting method stayed relatively consistent with the automated method.

In this specification and the appended claims, the singular forms "a," "an," and "the" include plural reference, unless the context clearly dictates otherwise.

## Claims

1. A method for simultaneously determining yeast budding status and viability of yeast cells in a sample, consisting of:
staining a sample to be analyzed with a first dye and with a second dye in a buffer solution;
acquiring a first fluorescent image of the sample stained with the first and second dyes, the first fluorescent image corresponding to the fluorescence from the first dye;
acquiring a second fluorescent image of the sample stained with the first and second dyes, the second fluorescent image corresponding to the fluorescence from the second dye;
analyzing the first fluorescent image to determine the aspect ratio of yeast cells by a computer-based automated process, thereby determining the yeast budding status of budding yeast cells in the sample; and
analyzing the second fluorescent image to determine viability of yeast cells in the sample, wherein the first dye is selected from the group consisting of Acridine Orange, SYTO 9, DAPI, Hoechst, Calcofluor White and the second dye is selected from the group consisting of Propidium Iodide, Ethidium Bromide, Oxonol, Mg-ANS.

2. The method of Claim 1, wherein the computer-based automated process comprises image analysis to measure the shape of budding yeasts.

3. The method of Claim 1, wherein a yeast cell is considered budding if its aspect ratio is 1.1 or greater.

4. The method of Claim 1, wherein the sample to be tested is a sample from a biofuel fermentation process.

5. The method of Claim 4, wherein the biofuel fermentation process comprises producing ethanol, butanol or methanol.

6. A method for simultaneously determining yeast budding status, viability, and concentration of yeast cells in a sample, consisting of:
staining a sample to be analyzed with a first dye and with a second dye in a buffer solution;
acquiring a first fluorescent image of the sample stained with the first and second dyes, the first fluorescent image corresponding to the fluorescence from the first dye;
acquiring a second fluorescent image of the sample stained with the first and second dyes, the second fluorescent image corresponding to the fluorescence from the second dye;
analyzing the first fluorescent image to determine the aspect ratio of yeast cells by a computer-based automated process, thereby determining the yeast budding status of budding yeast cells in the sample;
analyzing the second fluorescent image to determine viability of yeast cells in the sample; and
analyzing the first and second fluorescent images to determine the concentration of yeast cells in the sample, wherein the first dye is selected from the group consisting of Acridine Orange, SYTO 9, DAPI, Hoechst, Calcofluor White and the second dye is selected from the group consisting of Propidium Iodide, Ethidium Bromide, Oxonol, Mg-ANS.

## Patentansprüche

1. Verfahren zur gleichzeitigen Bestimmung des Hefeknospungsstatus und der Lebensfähigkeit von Hefezellen in einer Probe, bestehend aus:
Färben einer zu untersuchenden Probe mit einem ersten Farbstoff und mit einem zweiten Farbstoff in einer Pufferlösung;
Aufnehmen eines ersten Fluoreszenzbildes der mit dem ersten und zweiten Farbstoff gefärbten Probe, wobei das erste Fluoreszenzbild der Fluoreszenz des ersten Farbstoffs entspricht;
Aufnehmen eines zweiten Fluoreszenzbildes der mit dem ersten und dem zweiten Farbstoff gefärbten Probe, wobei das zweite Fluoreszenzbild der Fluoreszenz des zweiten Farbstoffs entspricht;
Analysieren des ersten Fluoreszenzbildes zur Bestimmung des Seitenverhältnisses von Hefezellen durch einen computerbasierten automatisierten Prozess, wodurch der Hefeknospungsstatus von Hefezellen in der Probe bestimmt wird; und
Analysieren des zweiten Fluoreszenzbildes, um die Lebensfähigkeit von Hefezellen in der Probe zu bestimmen, wobei der erste Farbstoff aus der Gruppe ausgewählt ist, die aus Acridinorange, SYTO 9, DAPI, Hoechst, Calcofluorweiß besteht, und der zweite Farbstoff aus der Gruppe ausgewählt ist, die aus Propidiumiodid, Ethidiumbromid, Oxonol, Mg-ANS besteht.

2. Verfahren nach Anspruch 1, wobei das computergestützte automatisierte Verfahren eine Bildanalyse zur Messung der Form von knospenden Hefen umfasst.

3. Verfahren nach Anspruch 1, wobei eine Hefezelle als knospend angesehen wird, wenn ihr Seitenverhältnis 1,1 oder größer ist.

4. Verfahren nach Anspruch 1, wobei die zu untersuchende Probe eine Probe aus einem Biokraftstoff-Fermentationsprozess ist.

5. Verfahren nach Anspruch 4, wobei der Biokraftstoff-Fermentationsprozess die Herstellung von Ethanol, Butanol oder Methanol umfasst.

6. Verfahren zur gleichzeitigen Bestimmung des Hefeknospungsstatus, der Lebensfähigkeit und der Konzentration von Hefezellen in einer Probe, bestehend aus:
Färben einer zu analysierenden Probe mit einem ersten Farbstoff und mit einem zweiten Farbstoff in einer Pufferlösung;
Aufnehmen eines ersten Fluoreszenzbildes der mit dem ersten und zweiten Farbstoff gefärbten Probe, wobei das erste Fluoreszenzbild der Fluoreszenz des ersten Farbstoffs entspricht;
Aufnehmen eines zweiten Fluoreszenzbildes der mit dem ersten und dem zweiten Farbstoff gefärbten Probe, wobei das zweite Fluoreszenzbild der Fluoreszenz des zweiten Farbstoffes entspricht;
Analysieren des ersten Fluoreszenzbildes, um das Seitenverhältnis der Hefezellen durch einen computerbasierten automatisierten Prozess zu bestimmen, wodurch der Hefeknospungsstatus der Hefezellen in der Probe bestimmt wird;
Analysieren des zweiten Fluoreszenzbildes, um die Lebensfähigkeit der Hefezellen in der Probe zu bestimmen, und
Analysieren des ersten und zweiten Fluoreszenzbildes, um die Konzentration von Hefezellen in der Probe zu bestimmen,
wobei der erste Farbstoff ausgewählt ist aus der Gruppe bestehend aus Acridinorange, SYTO 9, DAPI, Hoechst, Calcofluorweiß und der zweite Farbstoff ausgewählt ist aus der Gruppe bestehend aus Propidiumiodid, Ethidiumbromid, Oxonol, Mg-ANS.

## Revendications

1. Procédé pour déterminer simultanément l'état de bourgeonnement de levures et la viabilité des cellules de levure dans un échantillon, consistant en:
la coloration d'un échantillon à analyser avec un premier colorant et avec un second colorant dans une solution tampon ;
l'acquisition d'une première image fluorescente de l'échantillon coloré avec les premier et second colorants, la première image fluorescente correspondant à la fluorescence du premier colorant ;
l'acquisition d'une deuxième image fluorescente de l'échantillon coloré avec les premier et deuxième colorants, la deuxième image fluorescente correspondant à la fluorescence du deuxième colorant ;
l'analyse de la première image fluorescente pour déterminer le rapport d'aspect des cellules de levure par un processus automatisé par ordinateur, ainsi déterminant l'état de bourgeonnement des cellules de levure dans l'échantillon ; et
analyse de la deuxième image fluorescente pour déterminer la viabilité des cellules de levure dans l'échantillon, le premier colorant étant choisi dans le groupe constitué par l'Acridine Orange, le SYTO 9, le DAPI, le Hoechst, le Calcofluor White et le deuxième colorant étant choisi dans le groupe constitué par l'iodure de propidium, le bromure d'éthidium, l'Oxonol, le Mg-ANS.

2. Procédé de la revendication 1, dans lequel le processus automatisé par ordinateur comprend l'analyse d'images pour mesurer la forme des levures bourgeonnantes.

3. Procédé de la revendication 1, dans lequel une cellule de levure est considérée comme bourgeonnante si son rapport d'aspect est de 1,1 ou plus.

4. Procédé de la revendication 1, dans lequel l'échantillon à tester est un échantillon provenant d'un processus de fermentation de biocarburant.

5. Le procédé de la revendication 4, dans lequel le processus de fermentation de biocarburant comprend la production d'éthanol, de butanol ou de méthanol.

6. Procédé pour déterminer simultanément l'état de bourgeonnement de levures, la viabilité et la concentration des cellules de levure dans un échantillon, consistant en:
la coloration d'un échantillon à analyser avec un premier colorant et avec un second colorant dans une solution tampon ;
l'acquisition d'une première image fluorescente de l'échantillon coloré avec les premier et second colorants, la première image fluorescente correspondant à la fluorescence du premier colorant ;
l'acquisition d'une deuxième image fluorescente de l'échantillon coloré avec les premier et deuxième colorants, la deuxième image fluorescente correspondant à la fluorescence du deuxième colorant ;
l'analyse de la première image fluorescente pour déterminer le rapport d'aspect des cellules de levure par un processus automatisé par ordinateur, ainsi déterminant l'état de bourgeonnement des cellules de levure dans l'échantillon ;
l'analyse de la deuxième image fluorescente pour déterminer la viabilité des cellules de levure dans l'échantillon, et
l'analyse des première et deuxième images fluorescentes pour déterminer la concentration de cellules de levure dans l'échantillon,
le premier colorant étant choisi dans le groupe constitué par Acridine Orange, SYTO 9, DAPI, Hoechst, Calcofluor White et le second colorant étant choisi dans le groupe constitué par Iodure de Propidium, Bromure d'Ethidium, Oxonol, Mg-ANS.
